Europäisches Patentamt

European Patent Office

Office européen des brevets

(11) Veröffentlichungsnummer: **0 279 294**
**A1**

(19)

## (12) EUROPÄISCHE PATENTANMELDUNG

(21) Anmeldenummer: 88101637.2

(22) Anmeldetag: 04.02.88

(51) Int. Cl.⁴: **A61B 6/14** , A61B 6/03 ,
G01T 1/00

(30) Priorität: 16.02.87 DE 3704858

(43) Veröffentlichungstag der Anmeldung:
24.08.88 Patentblatt 88/34

(84) Benannte Vertragsstaaten:
DE FR GB IT SE

(71) Anmelder: **Siemens Aktiengesellschaft Berlin und München**
**Wittelsbacherplatz 2**
**D-8000 München 2(DE)**

(72) Erfinder: **Pfeiffer, Joachim, Dr.**
**Eifelstrasse 33**
**D-6140 Bensheim(DE)**
Erfinder: **Günther, Werner**
**Odenwaldstrasse 20**
**D-6140 Bensheim(DE)**
Erfinder: **Müther, Manfred**
**Adolf-Kolping-Strasse 20**
**D-6140 Bensheim(DE)**
Erfinder: **Heubeck, Erich**
**Blütenweg 11**
**D-6140 Bensheim(DE)**

(54) **Zahnärztliche Röntgendiagnostikeinrichtung zur Erstellung von Panorama-Schichtaufnahmen vom Kiefer eines Patienten.**

(57) Die Einrichtung enthält anstelle eines Röntgenfilms eine Detektoranordnung (11), welche elektrische Signale proportional zur Strahlungsintensität bildet. An die Detektoranordnung schließen sich ein A/D-Wandler (16), ein Bildspeicher (18) sowie eine Datenverarbeitungseinrichtung mit Rechner (21) an, welcher aus den von der Detektoranordnung während eines Aufnahmeablaufs gelieferten Signalen ein Übersichtsbild berechnet. Die Detektoranordnung enthält ein oder mehrere CCD-Sensoren (11, 12, 13), die so angeordnet sind, daß sich auf deren Bildzone (11a) der Sekundärspalt (8) abbilden läßt. Mit Hilfe eines Taktgenerators (22) werden den Lichtbildern (LiB) im Sekundärspalt (8) entsprechende Ladungsbilder (LB) im CCD-Sensor zu einem Gesamtladungsbild aufaddiert, wobei die Ladungsbilder (LB) aus der Bildzone (11a) in eine Speicherzone (11b) transportiert, danach über ein shift-Register (11c) ausgelesen und sodann in den A/D-Wandler (16) gegeben werden. Die Taktfrequenz ($f_{Takt}$) ist so gewählt, daß das Ladungsbild (LB) mit der gleichen Geschwindigkeit (v) in die Speicherzone getaktet und dann zeilenweise über das shift-Register ausgetaktet wird, mit der ein Röntgenfilm bei konventioneller Aufnahmetechnik relativ zum Sekundärspalt (8) bewegt wird.

FIG 4

# Zahnärztliche Röntgendiagnostikeinrichtung zur Erstellung von Panorama-Schichtaufnahmen vom Kiefer eines Patienten

Die Erfindung bezieht sich auf eine zahnärztliche Röntgendiagnostikeinrichtung zur Erstellung von Panorama-Schichtaufnahmen vom Kiefer eines Patienten, enthaltend eine um eine vertikale Achse drehbare Einheit, die Träger einerseits einer Röntgenstrahlenquelle und andererseits einer Blende mit Sekundärspalt ist, enthaltend ferner Mittel zur Konversion der Röntgenstrahlen in sichtbare Strahlen, eine Detektoranordnung, welche elektrische Signale proportional zur Strahlungsintensität bildet, einem mit der Detektoranordnung verbundenen A/D-Wandler, der die von der Detektoranordnung gewonnenen Spannungen digitalisiert, einen Bildspei-Bildspeicher sowie eine Datenverarbeitungseinrichtung mit Rechner, welche aus den von der Detektoranordnung während eines Aufnahmeablaufs gelieferten Signalen ein Übersichtsbild berechnet, welches über eine an die Datenverarbeitungseinrichtung angeschlossene Bildwiedergabeeinrichtung wiedergegeben werden kann.

Eine Einrichtung dieser Art ist beispielsweise aus der DE-PS 26 46 638 bekannt. Gegenüber konventioneller Aufnahmetechnik mit Röntgenfilm kann bei dieser bekannten Einrichtung eine Röntgenaufnahme elektronisch aufgezeichnet und abgespeichert und schließlich auf einem Bildschirm wiedergegeben werden. Bei der bekannten Einrichtung ist der zur Anwendung vorgesehene Detektor nicht weiter beschrieben.

Der im Patentanspruch 1 angegebenen Erfindung liegt die Aufgabe zugrunde, demgegenüber eine konstruktive Lösung aufzuzeigen, mit der unter Zugrundelegung verfügbarer Bildaufnehmer auf elektronischem Wege ein Panorama-Schichtbild, also ein Bild vom gesamten Kiefer eines Patienten, erhalten werden kann.

Vorteilhafte Ausgestaltungen und Weiterbildungen der Erfindung sind in den Unteransprüchen enthalten.

Die Erfindung wird nachfolgend anhand von Ausführungsbeispielen näher erläutert. Es zeigen:

Figur 1 ein Übersichtsbild mit den wesentlichen Teilen einer zahnärztlichen Röntgendiagnostikeinrichtung gemäß der Erfindung,

Figur 2 einen Teil der Strahlenaufnahmeeinheit in schaubildlicher Darstellung,

Figur 3 eine Variante zu der in Figur 2 dargestellten Ausführung,

Figur 4 ein Prinzipschaltbild,

Figuren 5 und 6 eine vereinfachte Darstellung von Sekundärspalt einerseits und Bildaufnahmesensor andererseits zur Erläuterung der Bildentstehung,

Figur 7 eine Diagrammfolge zur Erläuterung der Bildentstehung,

Figur 8 eine Prinzipdarstellung für eine Bildformatwandlung,

Figur 9 einen Ausschnitt von Figur 8 in vergrößerter, schaubildlicher Darstellung.

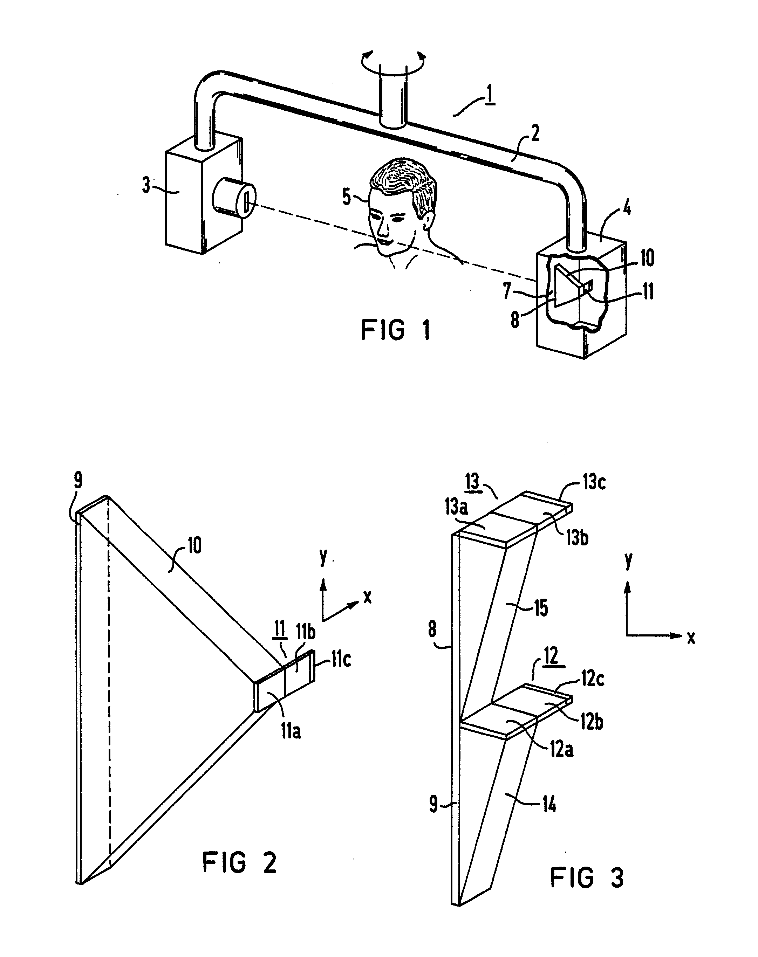

Die Figur 1 zeigt in stark vereinfachter perspektivischer Darstellung die wesentlichen Teile einer zahnärztlichen Röntgendiagnostikeinrichtung zur Erstellung von Panorama-Schichtaufnahmen. In bekannter Weise enthält die Diagnostikeinrichtung eine um eine lotrechte Achse schwenkbare Dreheinheit 1, bestehend aus einem Tragarm 2, an dessen einem Ende ein Röntgenstrahler 3 und an dessen anderem Ende eine Strahlenaufnahmeeinheit 4 gehaltert sind. Die Dreheinheit 1 ist in bekannter Weise um den Patientenkopf 5 herum - schwenkbar, wodurch sich eine Übersichtsaufnahme vom gesamten Kiefer 6 eines Patienten erstellen läßt.

Die Strahlenaufnahmeeinheit 4 enthält in bekannter Weise eine Blende 7 mit einem Sekundärspalt 8 in den Abmessungen von beispielsweise 5 × 125 mm. An den Sekundärspalt 8 - schließen sich Mittel zur Konversion der Röntgenstrahlen in sichtbare Strahlen an. Im vorliegenden Ausführungsbeispiel ist hierzu (Figur 2) in Sekundärspaltebene eine Szintillatorschicht 9 in den Abmessungen des Sekundärspaltes vorgesehen. An die Szintillatorschicht 9 schließt sich ein Bildübertragungselement 10 in Form einer Faseroptik an, die das Format des Sekundärspaltes von 5 × 125 mm auf das Format der Bildzone eines CCD-Sensors 11 mit den Abmessungen von beispielsweise 8 × 8 mm verkleinert.

Bei dem CCD-Sensor 11 handelt es sich um einen Typ, bei dem Speicher-und Bildzone (11a, 11b) innerhalb des chips räumlich getrennt angeordnet sind und bei dem das shift-Register 11b mit der CCD-Speicherzone 11c gekoppelt ist. Anstelle dieses CCD-Typs kann auch ein CCD-Typ verwendet werden, bei dem Bild und Speicherzone zusammenliegend innerhalb eines chips angeordnet sind.

Um die Funktionsbeschreibung zu vereinfachen, wird in der nachfolgenden Beschreibung davon ausgegangen, daß der gesamte Sekundärspalt 8 auf der Oberfläche eines einzigen CCD-Sensors abgebildet wird, wie dies in Figur 2 dargestellt ist. Aus heutiger Sicht ist es jedoch angezeigt, mehrere CCD-Sensoren vorzusehen, um den Sekundärspalt 8 abzudecken, wie dies in Figur 3 dargestellt ist. Entsprechend dieser Darstellung sind zur Abbildung des Sekundärspaltes 8 zwei etwa rechtwinkelig zur Sekundärspaltebene angeordnete CCD-Sensoren 12 und 13 und, als Übertragungselemente, zwei faseroptische Elemente 14, 15 vorgesehen. Die einzelnen CCD-Sensoren sind gleichen Typs, wie in Figur 2, also mit von der Bildzone räumlich getrennter Speicherzone und anschließendem shift-Register.

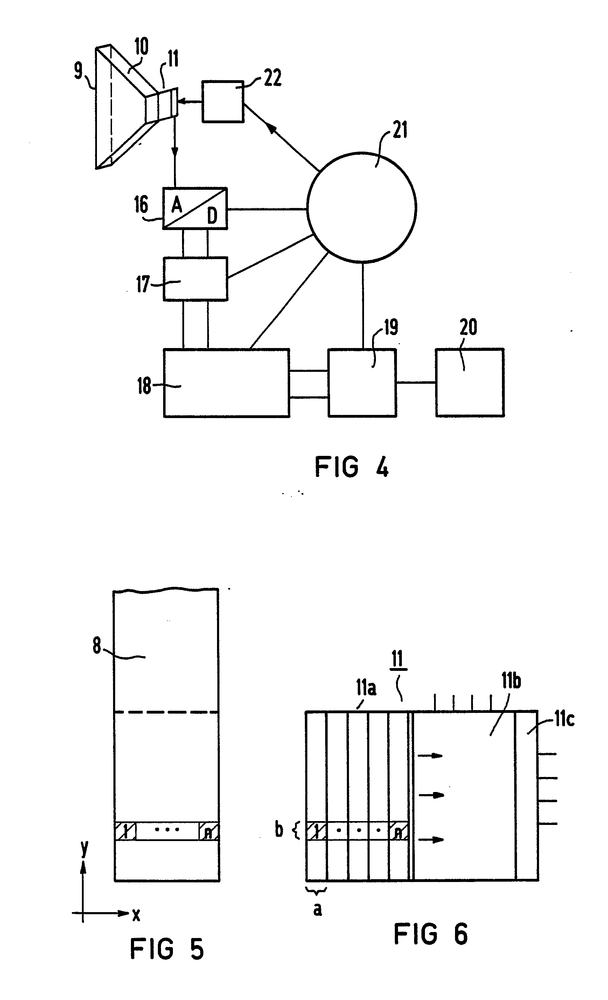

Die Signalverarbeitung wird anhand des Prinzipschaltbildes nach Figur 4 erläutert. Danach werden die aus der Detektoranordnung (CCD-Sensor/Sensoren) gewonnenen Signale an einen A/D-Wandler 16 gegeben, an den sich ein digitales Bildverarbeitungssystem mit einer Vorverarbeitungseinheit 17, einem Bildspeicher 18, einer Bildausleseeinheit 19, einem Monitor 20 und einem Rechner 21 anschließen. Mit 22 ist schließlich ein später noch näher erläuterter Taktgenerator bezeichnet, mit dem die CCD-Sensoren angesteuert werden.

Die am Ausgang des shift-Registers 11c des CCD-Sensors 11 Fig. 2) anliegenden Bildinformationen werden im A/D-Wandler digitalisiert und sodann entweder direkt in den Bildspeicher 18 eingegeben oder nach Vorverarbeitung in der Einheit 17 im Bildspeicher 18 abgespeichert. Der Rechner 21 gibt hierzu die notwendigen Steuer- bzw. Auslesebefehle.

Die direkte Eingabe der vom A/D-Wandler erhaltenen Signale in den Bildspeicher 18 ist dann vorteilhaft, wenn letzterer bei vertretbarem Kostenaufwand bzw. Bauvolumen eine entsprechend große Kapazität aufweist. In diesem Falle werden die Daten während eines Umlaufs der Dreheinheit zunächst nur abgespeichert und erst nach Abschluß der Aufnahme, also nach Umlauf des Strahlers um den Patientenkopf, verarbeitet, d.h. in der einer Filmaufnahme entsprechenden Geschwindigkeit aufaddiert. Wenngleich hierzu eine relativ große Datenmenge verarbeitet werden muß, so hat diese Lösung den Vorteil, daß eine Darstellung mehrerer beliebiger Schichten möglich ist.

Ist die unter vorgenanntem Gesichtspunkt notwendige Bildspeicherkapazität nicht mit wirtschaftlich vertretbarem Aufwand zu erhalten, so kann es vorteilhaft sein, die besagte Vorverarbeitungseinheit 17 zwischenzuschalten. Diese Vorverarbeitungseinheit enthält einen Zwischenspeicher und einen Arithmetikprozessor, wodurch die digitalen Daten aus dem A/D-Wandler entsprechend einem Steuerbefehl aus dem Rechner 21 zeitgerecht addiert, d.h. so addiert werden, daß ein Schichtbild einer gewünschten Schichtlage entsteht. Die hieraus resultierenden, aufgearbeiteten Daten werden anschließend in den Bildspeicher 18 gegeben. Durch die Zwischenschaltung der Vorverarbeitungseinheit 17 kann also ein Bildspeicher mit kleinerer Kapazität verwendet werden. Allerdings ist die Schichtlage durch Zwischenschalten einer solchen Vorverarbeitungseinheit festgelegt, d.h. die Schichtlage kann nicht mehr in gewissen Grenzen verändert werden, im Gegensatz zu der vorerwähnten Alternativanordnung ohne Vorverarbeitungseinheit, wo die Bilddaten erst nach einer Aufnahme zu einem Schichtbild zusammengesetzt werden.

Zwischen beiden Möglichkeiten kann auch ein Kompromiß vorgesehen sein, gemäß dem in der Vorverarbeitungseinheit 17 vor der Abspeicherung in den Bildspeicher mehrere benachbarte Bildspalten zeitgerecht aufaddiert werden und die Addition dieser Summenspalten zu einer Bildspalte nach Abspeicherung erfolgt.

Nachstehend wird anhand der Figuren 5 bis 7 die Bildentstehung erläutert:
Der Patient wird von einem durch eine im Röntgenstrahler 3 befindliche Primärblende begrenzten rechteckigen spaltförmigen Strahlenbündel durchstrahlt. Der Strahl trifft auf den Sekundärspalt 8 der Sekundärblende 7 und trifft sodann auf die Szintillatorschicht 9, wird dort in sichtbare Strahlen umgewandelt, die schließlich vom CCD-Sensor aufgenommen werden. Die vom CCD-Sensor aufgenommenen Signale sind proportional der Strahlungsintensität der dem Patientenkopf durchdringenden Röntgenstrahlung.

Bei konventioneller Schichtaufnahmetechnik wird hinter der Sekundärblende, also hinter dem Sekundärspalt 8, ein zu belichtender Röntgenfilm mit einer bestimmten Geschwindigkeit vorbeigezogen, wobei die gewählte Geschwindigkeit die Lage der tomographischen Schicht beeinflußt, d.h. durch Änderung der Filmgeschwindigkeit kann die tomographische Schicht geändert werden: Im vorliegenden Falle, wo der Röntgenfilm durch eine elektronische Detektoranordnung ersetzt wird, werden die am Detektor entstehenden Signale, wie nachfolgend näher erläutert, in bestimmter Weise zu einem auf einem Monitor wiedergebbaren Panorama-Schichtbild verarbeitet.

Anhand der Figuren 5 und 6 wird zunächst die Beziehung zwischen Sekundärspalt und der Bildzone des CCD-Sensors erläutert. Es wird davon ausgegangen, daß der Sekundärspalt 8 auf der Bildzone eines oder mehrerer CCD-Sensoren abgebildet wird. Das Abbildungsverhältnis in x-Richtung, also senkrecht zur Längsausdehnung des Sekundärspaltes 8, beträgt dabei $1 : n_x$ und in y-Richtung $1 : n_y$. Nachdem der Sekundärspalt eine Breite von etwa 5 mm und die heute verwendbaren CCD-Sensoren eine Bildzonenbreite von 8 mm haben, beträgt im Betrachtungsfalle $n_x = 1$. Je nach Anzahl und Größe der verwendeten Bildsensoren kann der Abildungsmaßstab $n_y$ in Spaltlängsrichtung zwischen 1 und etwa 20 liegen.

Ein Bildpunkt (Pixel) auf der CCD-Bildzonenfläche mit den Abmessungen a • b (a = Zeilenabstand, b = Spaltenabstand) entspricht einem

Pixel von $(n_x \cdot a)$ $(n_y \cdot b)$ in der Sekundärspaltebene. In der vereinfachten Übersicht nach Figuren 5 und 6 entsprechen also 1 bis n Licht-Bildpunkte im Sekundärspalt 1 bis n Ladungs-Bildpunkten im CCD-Sensor. In Richtung der Längsausdehnung des Sekundärspaltes gilt Analoges. Demgemäß wird eine Linie in Richtung des Sekundärspaltes auf einer CCD-Zeile abgebildet.

Durch Anlegen entsprechender Taktimpulse über den Taktgenerator 22 wird das Bild aus der Bildzone 11a in die Speicherzone 11b transportiert und von dort über das shift-Register 11c ausgelesen und in den A/D-Wandler eingegeben. Bei Normalbetrieb, d.h. in der Standardtaktfolge eines CCD-Sensors beträgt die Bildintegrationszeit etwa 20 ms. Danach wird das Bild in die Speicherzone getaktet. Hierzu sind so viele Takte notwendig, wie der CCD-Sensor Zeilen hat. Unter Zugrundelegung eines CCD-Typs mit 300 Zeilen und eines Taktes von 2 $\mu$s ist die Bildzone also nach ca. 0,6 ms entleert und kann dann sofort ein neues Bild aufnehmen. Während bei konventioneller Schichtaufnahmetechnik der Film mit einer bestimmten Geschwindigkeit hinter dem Sekundärspalt vorbeibewegt wird, also während der Bewegung des Filmes die am Sekundärspalt befindliche Bildinformation über einen bestimmten Zeitraum auf dem Bild integriert wird, wird gemäß der Erfindung diese Integration der Bildinformation elektronisch imitiert, indem das am Szintillator entstehende Lichtbild auf der Oberfläche des CCD-Sensors abgebildet und das entstehende Ladungsbild in einer bestimmten Taktfolge aus der Bildzone in die Speicherzone getaktet und dann zeilenweise über das shift-Register ausgetaktet wird. Die Taktfolge ist dabei so gewählt, daß das Ladungsbild, bezogen auf die Sekundärspaltebene, die gleiche Geschwindigkeit in x-Richtung hat, die ein Film bei konventioneller Schichtaufnahmetechnik haben würde. Die ermittelte Taktfrequenz steht in folgender Beziehung zu der Äquivalent-Geschwindigkeit (v) eines Filmes:

$$f_{Takt} = \frac{v}{n_x \cdot a}$$

wobei $f_{Takt}$ die Anzahl der Zeilen pro Sekunde und $(n_x \cdot a)$ den CCD-Zeilenabstand, bezogen auf die Sekundärspaltebene, angeben. Bei einer typischen Filmgeschwindigkeit von 30 mm/s und einem Zeilenabstand von 20 $\mu$m und unter Zugrundelegung eines Abbildungsverhältnisses von 1:1 in x-Richtung würde sich eine Taktfrequenz von 1.500 Hz ergeben.

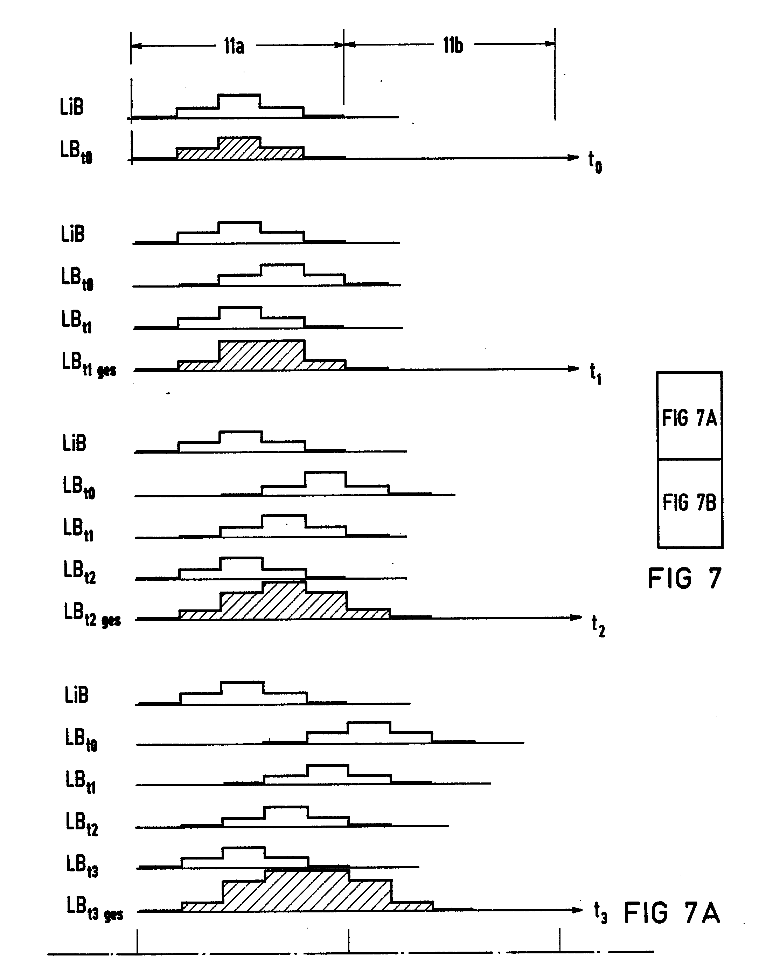

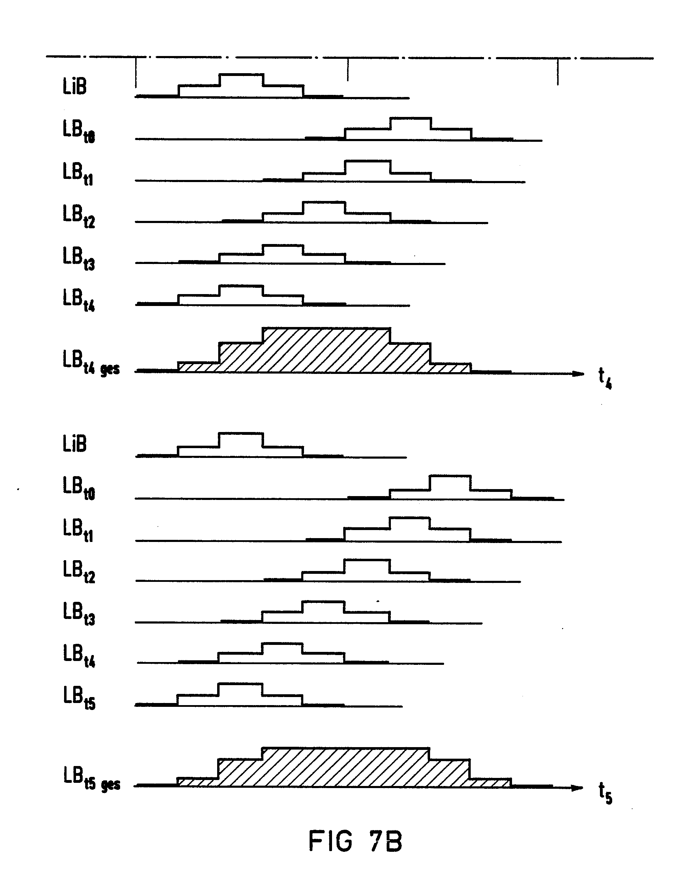

Der Vorgang der Bildintegration wird anhand der Diagrammfolgen entsprechend Figur 7 für fünf der in Figuren 5 und 6 angegebenen Bildpunkte 1

... n für die Zeitpunkte $t_0$ bis $t_5$ aufgezeigt. In den Diagrammen wird für fünf Bildpunkte mit unterschiedlicher Intensität (I) das Lichtbild (LiB) und darunter das zugehörige Ladungsbild (LB), wie es in der Bildzone des CCD-Sensors entsteht, aufgezeigt. Hieraus ergibt sich, daß zum Zeitpunkt $t_0$ für einen bestimmten "Licht-Bildpunkt 1" ein entsprechendes Ladungsbild vorliegt. Zum Zeitpunkt $t_1$ wird dieses Ladungsbild ($LB_{t0}$) in x-Richtung, also in Spaltrichtung des CCD-Sensors geschoben. Gleichzeitig wird wieder ein neuer Licht-Bildpunkt zum Ladungsbildpunkt ($LB_{t1}$). Zum Zeitpunkt $t_2$ werden dann wiederum die Ladungsbilder in x-Richtung verschoben und sofort. Die Integration der einzelnen Ladungsbilder ($LB_{t0}$, $LB_{t1}$ ...) ergibt das jeweils darunter gezeichnete Gesamtladungsbild ($LB_{t1ges}$, $LB_{t2ges}$ ...) für den betreffenden Zeitpunkt ($t_1$ ... $t_5$) betrachtet. Aus der Betrachtung ist erkennbar, daß in den Ladungsbildern demnach Licht-Bilder, die nacheinander (zeitlich versetzt) am gleichen Ort (Sekundärspalt) vorliegen, örtlich versetzt und aufaddiert werden. Dieser Vorgang entspricht genau dem Verhalten eines bewegten Filmes, wie es bei der bisherigen Schichtbildaufnahmetechnik vorgesehen wird. Die örtlich versetzte Addition der zeitlich aufeinanderfolgenden Licht-Bilder im Ladungsbild setzt sich fort, bis die betrachtete CCD-Zeile die Speicherzone erreicht hat (siehe Ladungsbild $LB_{t0}$ zum Zeitpunkt $t_5$. Erreicht die Zeile das shiftRegister (11c), so wird diese seriell ausgetaktet, A/D gewandelt und dann in den Bildspeicher als Spalte eingelesen.

Denkbar ist es auch, die Ladung mehrerer CCD-Zeilen aufzuaddieren und die Summe als Spalte in den Bildspeicher abzulegen (siehe hierzu die Anmerkungen bezüglich der Vorverarbeitungseinheit 17).

Eine zweite Möglichkeit, das Integrationsverhalten eines am Sekundärspalt vorbeiziehenden Filmes auf elektronischem Wege mittels eines CCD-Sensors zu imitieren, ist folgende:
Die Standardtaktfolge eines CCD-Sensors, die üblicherweise 15 kHz beträgt, wird dahingehend modifiziert, daß während der Bildintegrationszeit ($t_{BA}$) des CCD-Elements von z.B. 20 ms die Zeilen mit einer Taktrate ($f_{Takt}$) in der Bildzone verschoben werden, die der Filmgeschwindigkeit in konventioneller Aufnahmetechnik entspricht. Während der Bildintegrationszeit ($t_{BA}$) wandert das Ladungsbild innerhalb der Bildzone zwischen z.B. 0,1 und 1 mm. Nach der Bildintegrationszeit ($t_{BA}$) wird das gesamte Ladungsbild mit höchster Taktrate, die das CCD-Element verträgt, in die Speicherzone geschoben. Dies dauert etwa 0,6 ms bei etwa 300 CCD-Zeilen und einer Taktzeit von 2$\mu$s. Danach beginnt die nächste Bildaufnahmezeit. Während dieser Aufnahmezeit wird das Bild in der Speicherzone ausgetaktet und die digitalisierte Information in den Bildspeicher entsprechend versetzt einaddiert. Bild-und Speicherzonen können dabei unterschiedlich getaktet werden.

Der Zeitablaufplan sieht wie folgt aus:

Zum Zeitpunkt $t_0$ ist die Bildzone leer. An der Bildzone liegt ein Takt mit der Frequenz

$$f_{Takt} = \frac{v}{n_x \cdot a}$$

an. Während der Bildintegrationszeit $t_{BA}$ wird das Bild aus der Bildzone in die Speicherzone getaktet. Dabei vergeht die Zeit $t_v$ (beispielsweise 0,6 ms). Nach dem Austakten des Bildes aus der Bildzone in die Speicherzone ist die Bildzone leer und an der Speicherzone wird ein Takt zum Austakten der Information angelegt. Diese Taktfrequenz ist hier sehr hoch; sie kann z.B. 15.000 Zeilen/s betragen. Beim Austakten werden jeweils

$$\frac{P_x}{n_x \cdot a}$$

Zeilen aufsummiert, wobei $P_x$ die Ausdehnung eines Bildspeicherpixels in x-Richtung ist, bezogen auf die Sekundärspaltebene. Diese Summenzeile wird als Spalte im Bildspeicher abgelegt. Dieser Vorgang wiederholt sich im Weiteren periodisch.

Ein Bild, so wie es während einer jeden Bildintegrationszeit $t_{BA}$ ausgegeben wird, belegt im Bildspeicher

$$Z \cdot n_x \cdot \frac{a}{P_x}$$

Spalten, wobei Z ein Maß für die CCD-Zeilenzahl ist. Die Addition der Einzelbilder im Bildspeicher erfolgt versetzt dazu, wobei hier gilt:

$$m_{BSS} = \frac{v \cdot (t_{BA} + t_v)}{P_x}$$

wobei $m_{BSS}$ der Versatz in der Bildspeicherspaltennummer ist, um den versetzt zwei aufeinanderfolgende Bilder einsortiert werden.

Ein besonderer Vorteil der zuletzt aufgezeigten Möglichkeit im Vergleich zur ersten ist, daß man damit Störungen vermeiden kann, die durch den Dunkelstromeffekt (Ansammeln von nicht durch Licht verursachte Ladungen in der Ladungszone während der Entstehung des Ladungsbildes) hervorgerufen werden.

Wie bereits erwähnt, ist es beim heutigen Stand der Technik noch nicht möglich, einen Detektor zu bekommen, der die üblichen Abmessungen des Sekundärspaltes (5 $\times$ 120 mm) von Panorama-Schichtgeräten abdecken würde. Handelsübliche und dadurch relativ preiswerte CCD-Bildaufnehmer haben Formate in der Größenordnung 7 $\times$ 9 mm bei 400 bis 600 Bildpunkten (Pixeln). Bei den erwähnten Abmessungen des Sekundärspaltes von 5 $\times$ 120 mm und unter Zugrundelegung von 50 $\times$ 1200 Bildpunkten wäre es möglich, durch Anordnung zwei solcher Bildaufnehmer in den Abmessungen 7 $\times$ 9 mm den Sekundärspalt abzudecken. Hierzu ist es aber notwendig, das Format entsprechend zu wandeln.

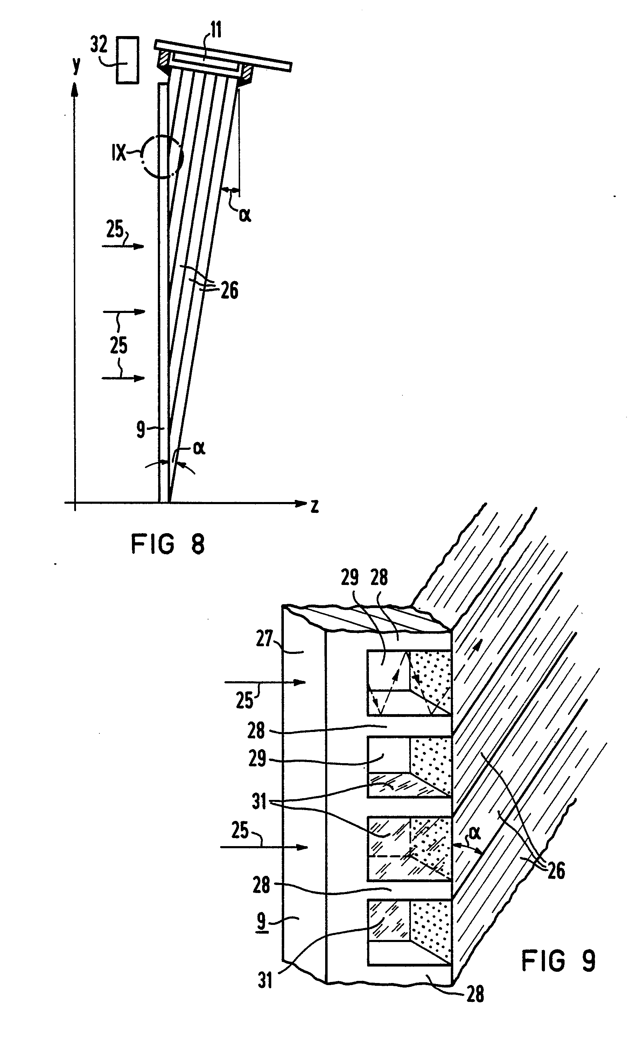

Die Figuren 8 und 9 zeigen den Aufbau eines Formatwandlers, wobei in Betrachtung der Figur 8 davon ausgegangen wird, daß, entsprechend der Prinzipdarstellung in Figur 2, das Format des Sekundärspaltes auf einen einzigen CCD-Sensor abgebildet werden soll. Der nachfolgend beschriebene Aufbau gilt analog auch für eine Anordnung entsprechend Figur 3, wo eine Abbildung des Sekundärspaltes auf mehrere CCD-Sensoren aufgeteilt ist.

Bei einer Röntgenbestrahlung (durch Pfeile 25 angedeutet) leuchtet das Szintillatormaterial der Szintillatorschicht 9, wobei ein Teil der ausgelösten Photonen von den Fasern der zur Übertragung vorgesehenen Faseroptik aufgefangen und zum Bildaufnehmer geleitet wird. Die Faseroptik besteht hier aus einem Bündel von einzelnen Fasern 26, die unter einem Winkel $\alpha$ zum Faserverlauf bzw. zur Ebene der Szintillatorschicht 9 angeschliffen sind. Die Szintillatorfläche wird dabei in y-Richtung um den Faktor sinus $\alpha$ verkleinert auf dem Bildaufnehmer (CCD-Sensor 11) abgebildet. In x-Richtung (senkrecht zur Papierebene) bleibt diese Abbildung unverzerrt, da hier die Breite des CCD-Sensors der Spaltbreite entspricht. Für sinus $\alpha$ etwa 0,15 ergibt sich z.B. eine Wandlung des Formates von 60 mm auf 9 mm. Mit einem solchen Detektor kann also ein Spalt von 7 $\times$ 60 mm auf einem handelsüblichen CCD-Sensor von 7 $\times$ 9 mm Aufnahmefläche abgebildet werden. Setzt man zwei solcher CCD-Sensoren, wie in Figur 3 dargestellt, übereinander, so läßt sich damit der Sekundärspalt von üblichen Dental-Panorama-Schichtgeräten abdecken. Unter Beibehaltung dieses Prinzips lassen sich selbstverständlich auch andere Formate abdecken, z.B. wie bereits erläutert, mit einem oder auch mit drei oder vier Sensoren pro Spalt und einer geeigneten Wahl des Winkels $\alpha$.

Eventuell auftretende Schwierigkeiten durch Lichtverluste kann man dadurch beseitigen, daß man zwischen Bildaufnehmer und dem oberen Faseroptikende einen Bildverstärker schaltet, z.B. einen Nahfokus-Bildverstärker.

Die Auflösung des Systems ist, wie auch bei Film-/Foliensystemen, von der Dicke der Leuchtschicht abhängig. Dieser Effekt ist besonders in y-Richtung, also in Längsausdehnung des Spaltes, wichtig, da hier die effektive Dicke der Leuchtschicht um den Faktor $1/\sin \alpha$ größer ist als die reale Dicke. Um hier Abhilfe zu schaffen, ist, entsprechend einer vorteilhaften Ausgestaltung, die Szintillatorschicht 9 nicht homogen, sondern in eine Struktur entsprechend der Anordnung nach Figur 9 eingebettet. Die Szintillatoranordnung besteht hier aus einem Kamm-oder rechenartigen Träger, welcher zwischen jeweils zwei Stegen 28 einen Raum 29 begrenzt, der mit Szintillatormaterial gefüllt ist. Der Träger 27 ist ca. $100 \mu m$ dick und besteht aus röntgenstrahlendurchlässigem Material, z.B. aus Aluminium, Kunststoff oder ähnlichem. Die den Röntgenstrahlen abgewandten Flächen 30 der Räume werden in gutem optischem Kontakt mit den Stirnseiten der Fasern 26 gebracht, die übrigen Begrenzungsflächen 31 können verspiegelt oder anderweitig reflektierend ausgebildet sein. Durch die vorgesehene Anordnung ist es möglich, die optischen Fasern nahezu um 90° aus der y-Richtung in z-Richtung umzulenken.

Damit der CCD-Sensor 11 vor Röntgenbestrahlung geschützt ist, ist zweckmäßigerweise ein entsprechender Bleischutz 32 vorgesehen.

**Ansprüche**

1. Zahnärztliche Röntgendiagnostikeinrichtung zur Erstellung von Panorama-Schichtaufnahmen vom Kiefer eines Patienten, enthaltend eine um eine vertikale Achse drehbare Einheit (1), die Träger einerseits einer Röntgenstrahlenquelle (3) und andererseits einer Blende(7) mit Sekundärspalt ist, enthaltend ferner Mittel (9) zur Konversion der Röntgenstrahlen in sichtbare Strahlen, eine Detektoranordnung (11), welche elektrische Signale proportional zur Strahlungsintensität bildet, enthaltend ferner einen mit der Detektoranordnung verbundenen A/D-Wandler (16), der die von der Detektoranordnung gewonnenen Spannungen digitalisiert, einen Bildspeicher (18) sowie eine Datenverarbeitungseinrichtung mit Rechner (21), welche aus den von der Detektoranordnung während eines Aufnahmeablaufs gelieferten Signalen ein Übersichtsbild berechnet, welches über eine an die Datenverarbeitungseinrichtung angeschlossene Bildwiedergabeeinrichtung (20) wiedergegeben werden kann, **dadurch gekennzeichnet,** daß die Detektoranordnung ein oder mehrere CCD-Sensoren (11, 12, 13) enthält, die so angeordnet sind, daß sich auf deren Bildzone (11a) der Sekundärspalt (8) abbilden läßt, wobei eine Linie in Richtung der Längsausdehnung des Spaltes auf einer CCD-Zeile abgebildet wird, daß ein mit dem CCD-Sensor verbundener Taktgenerator (22) vorhanden ist, durch dessen Taktimpulse Lichtbilder (LiB) im Sekundärspalt (8) entsprechende Ladungsbilder (LB) zu einem Gesamtladungsbild (LBges.) aufaddiert werden, wobei die Ladungsbilder (LB) aus der Bildzone (11a) in eine Speicherzone (11b) transportiert und danach über ein shift-Register (11c) ausgelesen und sodann in den A/D-Wandler (16) gegeben werden, und daß die Taktfrequenz ($f_{Takt}$) so gewählt ist, daß das Ladungsbild (LB) mit der gleichen Geschwindigkeit (v) in die Speicherzone getaktet und dann zeilenweise über das shift-Register ausgetaktet wird, mit der ein Röntgenfilm bei konventioneller Aufnahmetechnik relativ zum Sekundärspalt (8) bewegt wird.

2. Röntgeneinrichtung nach Anspruch 1, **dadurch gekennzeichnet,** daß die Taktfrequenz ($f_{Takt}$) nach der Beziehung

$$f_{Takt} = \frac{v}{n_x \cdot a}$$

gewählt ist.

3. Röntgeneinrichtung nach Anspruch 2, **dadurch gekennzeichnet,** daß die Ladung mehrerer CCD-Zeilen aufaddiert wird und die Summe dieser integrierten Ladung sodann im Bildspeicher (18) abgelegt wird.

4. Röntgeneinrichtung nach Anspruch 1, **dadurch gekennzeichnet,** daß während der Bildintegrationszeit ($t_{BA}$) des CCD-Sensors (11) die Bildzeilen mit einer Taktrate ($t_{Takt}$) in der Bildzone (11a) verschoben werden, die der Filmgeschwindigkeit (v) bei konventioneller Aufnahmetechnik entspricht, und daß nach der Bildintegrationszeit ($t_{BA}$) das gesamte Ladungsbild mit einer höheren Taktrate in die Speicherzone (11b) geschoben wird.

5. Röntgeneinrichtung nach Anspruch 1, **dadurch gekennzeichnet,** daß mehrere, über die Länge des Sekundärspaltes (8) verteilt angeordnete CCD-Sensoren (11, 12, 13) vorgesehen sind, und diese so angeordnet sind, daß deren Bildzone (11a, 12a, 13a) unter einem Winkel ($\alpha$) ≧ 90° zur Ebene des Sekundärspaltes (8) verläuft und daß zwischen Sekundärspalt (8) und Bildzone (11a, 12a, 13a) ein Bildwandler (9) und eine Bildübertragungsoptik (26, 14, 15) vorgesehen sind.

6. Röntgeneinrichtung nach Anspruch 5, **dadurch gekennzeichnet,** daß zur Bildübertragung eine Faseroptik (26) vorgesehen ist, deren eines, auf die Bildzone (11a) gerichtetes Ende senkrecht und deren anderes, dem Sekundärspalt (8) zugewandtes Ende unter dem vorgenannten Winkel (α) zur Faserrichtung abschließt.

7. Röntgeneinrichtung nach Anspruch 6, **dadurch gekennzeichnet,** daß das dem Sekundärspalt (8) zugewandte Ende der Faseroptik (26) mit einem der Spaltgröße entsprechenden Körper (27) aus strahlendurchlässigem Material verbunden ist und der Körper (27) zumindest zur Faseroptik (26) hin offene Kammern (29) enthält, welche mit Szintillatormaterial ausgefüllt sind.

8. Röntgeneinrichtung nach Anspruch 7, **dadurch gekennzeichnet,** daß der Körper (27) kammförmig angeordnete dünne Stege (28) aufweist, wodurch in Spaltrichtung eine Vielzahl von übereinander angeordneten Kammern (29) gebildet werden.

9. Röntgeneinrichtung nach Anspruch 8, **dadurch gekennzeichnet,** daß die die Kammern (29) begrenzenden Innenflächen (30, 31) des Körpers (27) verspiegelt sind.

10. Röntgeneinrichtung nach einem der Ansprüche 1 bis 9, **dadurch gekennzeichnet,** daß CCD-Sensoren (11, 12, 13) mit von der Bildzone (11a, 12a, 13a) räumlich getrennter Speicherzone (11b, 12b, 13b) verwendet sind.

FIG 1

FIG 2

FIG 3

FIG 4

FIG 5

FIG 6

FIG 7A

FIG 7B

FIG 7

FIG 7A

人

FIG 7B

FIG 8

FIG 9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 138 625 (PICKER INT. INC.) * Figuren 1-12; Seite 7, Zeile 23 - Seite 15, Zeile 13; Seite 16, Zeile 11 - Seite 34, Zeile 10 * --- | 1,3-6 | A 61 B 6/14 A 61 B 6/03 G 01 T 1/00 |
| A | US-A-4 383 327 (KRUGER) * Figuren 1,2,4; Spalte 4, Zeile 1 - Spalte 5, Zeile 49; Spalte 6, Zeile 7 - Spalte 8, Zeile 38; Spalte 9, Zeile 49 - Spalte 12, Zeile 50 * --- | 1,3,4, 10 | |
| A | US-A-4 298 800 (GOLDMAN) * Spalte 6, Zeile 47 - Spalte 13, Zeile 43 * --- | 1,5,6 | |
| A | DE-A-3 141 755 (HITACHI MEDICAL CORP.) * Figuren 1-3; Seite 10, Zeile 24 - Seite 12, Zeile 16; Seite 14, Zeile 1 - Seite 20, Zeile 22 * --- | 7,9 | |
| A | REVIEW OF SCIENTIFIC INSTRUMENTS, Band 48, Nr. 6, Juni 1977, Seiten 669-672, American Institute of Physics, New York, US; L.N. KOPPEL: "Direct soft x-ray response of a charge-coupled image sensor" * Seite 669, "Introduction", Abschnitt "CCD video sensors"; Seiten 669,670, Abschnitte "SID detector" und "SID x-ray sensitivity"; Figur 2 * ----- | 10 | **RECHERCHIERTE SACHGEBIETE (Int. Cl.4)** A 61 B G 01 T |

Der vorliegende Recherchenbericht wurde für alle Patentansprüche erstellt

| Recherchenort | Abschlußdatum der Recherche | Prüfer |
|---|---|---|
| DEN HAAG | 19-05-1988 | CHEN A.H. |

EPO FORM 1503 03.82 (P0403)